# EUROPEAN PATENT SPECIFICATION

(11) **EP 4 757 706 B1**
(45) Date of publication and mention of the grant of the patent: **18.11.2026**
(21) Application number: 25741282.5
(22) Date of filing: 11.07.2025
(51) Int. Cl.: A61B 5/09, A61B 5/22, A61B 5/097, A61M 16/00, A63B 23/18

(54) **DEVICE FOR MEASURING THE INSPIRATION AND EXPIRATION MUSCLE STRENGTH OF A PATIENT**
VORRICHTUNG ZUR MESSUNG DER INSPIRATIONS- UND EXSPIRATIONSMUSKELSTÄRKE EINES PATIENTEN
DISPOSITIF DE MESURE DE LA FORCE MUSCULAIRE D'INSPIRATION ET D'EXPIRATION D'UN PATIENT

(30) Priority: 02.09.2024 IT 202400019543
(43) Date of publication of application: 17.06.2026
(73) Proprietor: MIR - MEDICAL INTERNATIONAL RESEARCH S.P.A., 00173 Roma (RM) (IT)
(72) Inventor: LUPI, Fabio, 00074 Nemi (RM) (IT); PENNACCHIETTI, Marco, 00159 Roma (RM) (IT); POMELLA, Luca, 00172 Roma (RM) (IT); ALLEGRI, Stefano, 00137 Roma (RM) (IT)
(74) Representative: Ripamonti, Enrico
(86) International application number: PCT/IB2025/057043
(87) International publication number: WO 2026/047421

(56) References cited:
- CN-A- 109 126 053
- KR-A- 20230 077 283
- US-A1- 2019 299 055
- US-A1- 2021 001 169

## Description

The present invention relates to a device adapted to evaluate the respiratory capacity of a patient, according to the preamble of the main claim.

In particular, the present invention relates to a device for testing the respiratory capacity of a person both for the purposes of approach adapted to improve a pulmonary function and for improving a respiratory capacity, for example in athletes, singers and the like. The persons considered in this latter case, will however be indicated as "patients" in the present document.

The device subject of the invention is in particular adapted to measure the inspiration and expiration muscle strength of a patient by measuring the pressure of the air inspired and/or expired by the patient. As a matter of fact, it is known that the measuring of the maximum static inspiration pressure that a person may generate at the mouth or the maximum static expiration pressure is a simple way for measuring the inspiration and expiration muscle strength: as a matter of fact, the pressure measured during these operations reflects the pressure developed by the respiratory muscles plus the passive elastic recoil pressure of the respiratory system comprising the lungs and the thoracic wall.

Historically, there was used an aneroid manometer for measuring the pressure mentioned above. However, such use entailed the generation of an analogue signal on a display which was difficult to read precisely; furthermore, in such detected signal, the pressure transients were difficult to remove.

However, there are known various devices (which we will refer to as "respiratory devices") adapted to allow an inspiration or expiration training for the patient. The oldest devices only allowed the actuation of one of the two evaluations that is of the maximum expired pressure or of the maximum inspired pressure. Subsequently, there were known devices adapted to allow both such measurements and specifically aimed at activating the respiratory muscles. Such devices are both for personal use and for clinical use.

Such prior art devices reveal various drawbacks which range from the impossibility to define the total respiratory capacity of the lungs in both phases (inspiration and expiration) to the excessive dimension (like in the case of devices for clinical use) which make them difficult to use. The latter devices are also not cost-effective.

US11759677 discloses a device adapted to evaluate both the inspiration and expiration functions, same case applying to an independent adjustment of the resistance levels of the airways during such functions, adjustment used when using the prior art device. This prior art device described in the United States prior art document is still significantly large in size and must be associated with a further member for evaluating the collected data which is large in size.

An object of the present invention is to provide a device of the type mentioned above which allows to measure the inspiration and expiration muscle strength, which overcomes the drawbacks of the known devices of the prior art.

In particular, an object of the present invention is to provide a device of the type indicated above that is small in size and which is easy to use for a user.

A further object of the present invention is to provide a device of the type mentioned above which also allows to evaluate the ability of a patient to carry out an inspiration operation referred to as "sniff", a brief and acute voluntary action carried out using one or both nostrils not occluded and which entails the contraction of the diaphragm and of other inspiratory muscles of the patient.

These and other objects which shall be apparent to the person skilled in the art are attained by a device for measuring the inspiration and expiration muscle strength of a user according to the attached claims.

For a better understanding of the present invention, attached hereto purely by way of non-limiting example are the following drawings, wherein:
figure 1 shows a perspective view of a device according to the present invention;

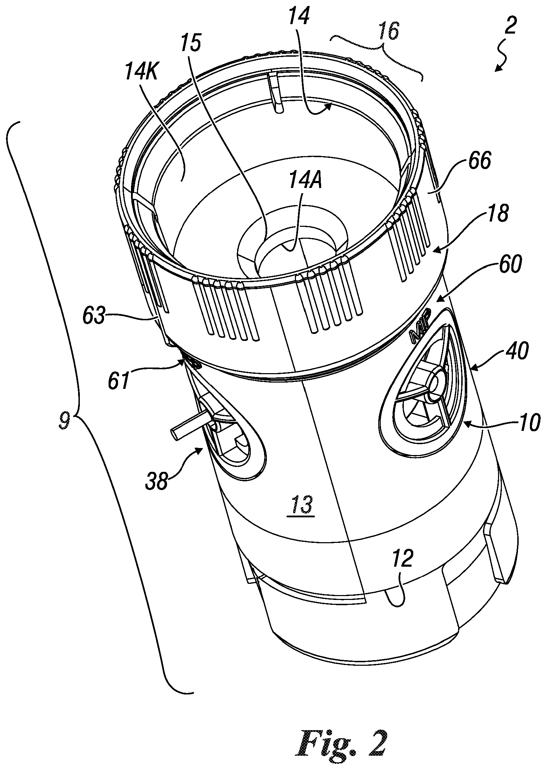

figure 2 shows a top perspective view of a component of the device of figure 1;

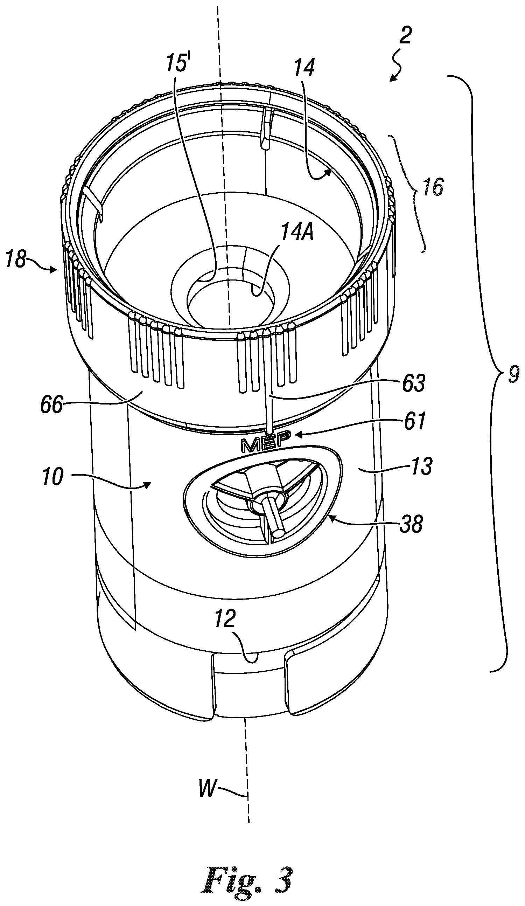

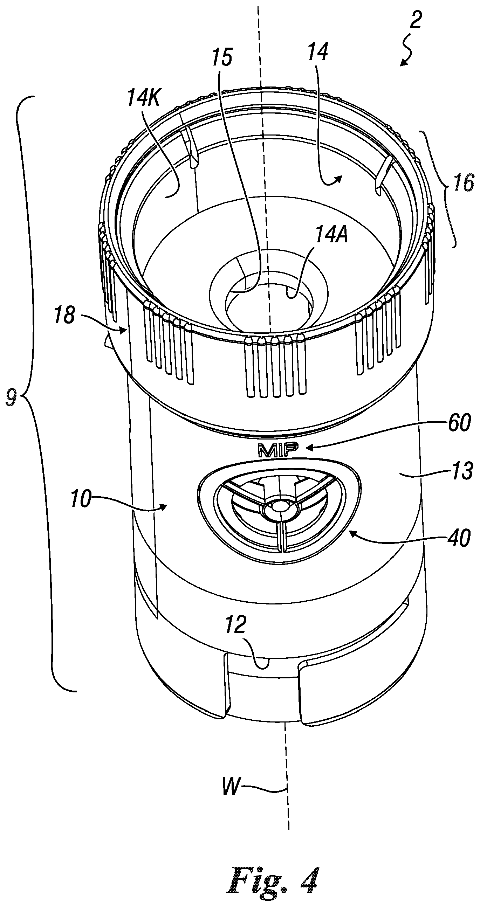

figures 3 and 4 show perspective views of the component of figure 2 from different angles;

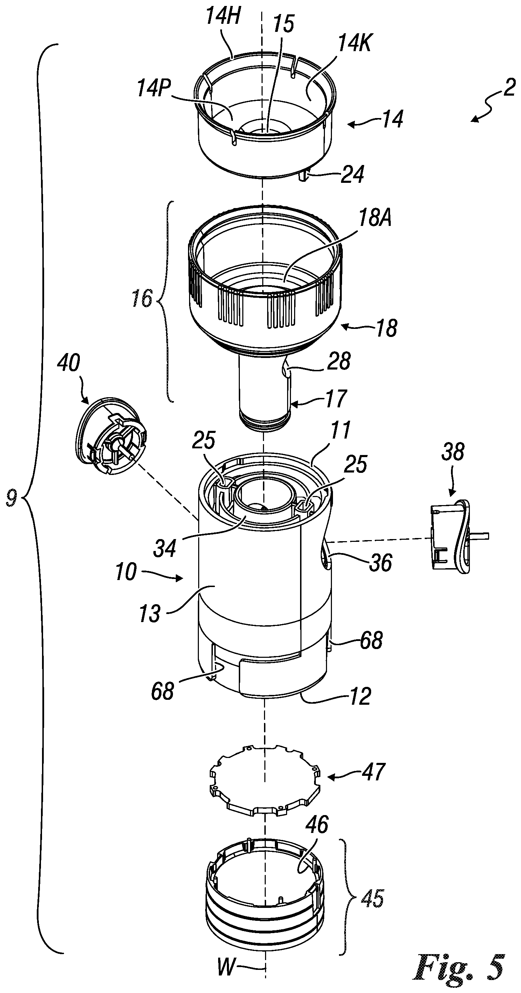

figure 5 shows a top exploded view of the component of figure 2;
figure 6 shows a bottom exploded view and from a different angle with respect to figure 5, of the component of figure 2;
figure 7 shows a top view of the component of figure 2; and
figure 8 shows a cross-sectional view according to line 8-8 of figure 7.

With reference to the figures mentioned above, a device according to the present invention is generally indicated with 1 and it comprises a first component 2 for measuring the pressure of the air inspired (MIP) and the pressure of the air expired (MEP) by a user or patient so as to evaluate the inspiration and expiration muscle strength of such patient. Such first component 2 is adapted to be inserted into a seat 3 of a second component 4 adapted to detect the datum of such measurement so as to be able to process it so as to evaluate the muscle strength mentioned above. This, with the methods described below.

Preferably and advantageously, such second component comprises a body 5 whose dimensions allow it to be held by only one hand of the patient when implementing the pressure measurement. In an embodiment, such body 5 may have a display 6 in which the measured datum is displayed.

Even more preferably and advantageously, such second component 4 is a medical product adapted to allow the implementation of a spirometer test (that is, it is a "spirometer") when a known turbine (not shown) that allows carry out said test is associated therewith. The turbine is adapted to be inserted into the seat 3 (through hole) containing the first component 2: at such seat or therein, there is arranged a usual detector (not shown) which is functionally associated with a detection element or RFID TAG. Such RFID TAG is static, it does not rotate through the action of the flow which flows through the turbine and which drives it in rotation and contains data relating to the use of the turbine. The detection of the rotation of such movable equipment by the detector allows carrying out the spirometer test.

A similar detection element is associated with the first component 2 to transfer the detected data by such component to the second component 4.

More particularly, the first component 2 has a body 9 comprising a tubular-shaped cylindrical element 10 provided with an inner cavity 10A opening at ends 11 and 12 and delimited by an outer wall 13. At the first end 11 (upper in the figures) there is arranged a drinking-glass-like element 14, open at the bottom part in a hole 15, adapted to be received in a switch element 16 having a tubular part 17 (at whose first end 17A there enters the a collar 14A of the drinking-glass-like element 14 protruding from a bottom 14P of such element in which the hole 15 is present).

The switch element 16 has a drinking-glass-like control portion 18 which houses the drinking-glass-like element 14 and from which there extends the tubular part 17. Such drinking-glass-like control portion 18 (or simply "drinking-glass-like portion") rests on the first end 11 of the element 10 and it can rotate thereon around a longitudinal axis W of the body 9.

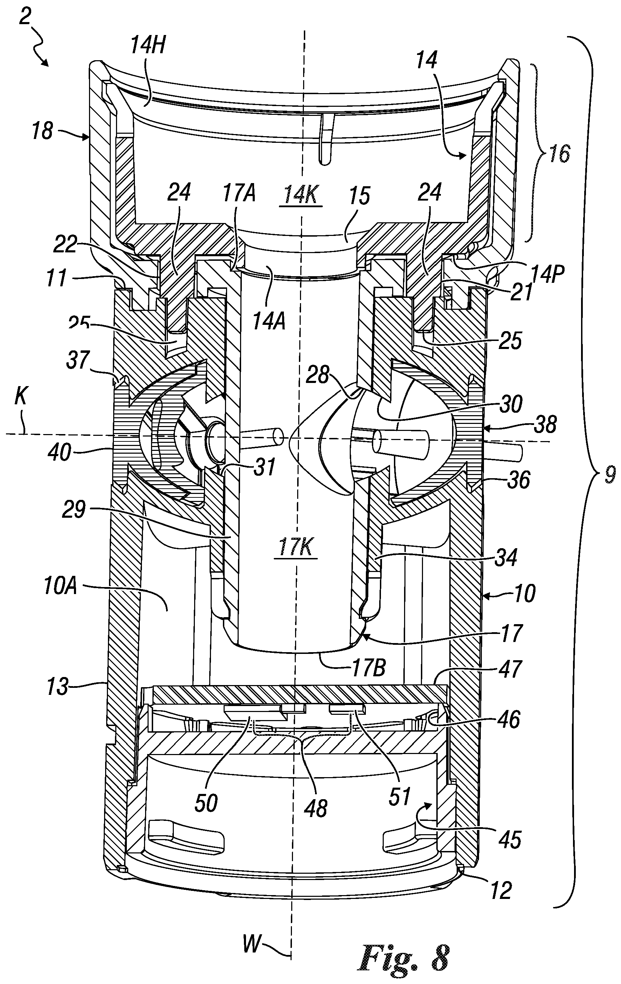

More particularly, the drinking-glass-like portion 18 of the switch element 16 has a bottom part 18A from whose face 20 there extends the tubular part 17. The latter has a hole 17K which opens on one side to its first end 17A and on the other side to its second end 17B in the inner cavity 10A of the element 10 mentioned above. In this manner, as observable in figure 8 for example, the hole 17 places such cavity 10A in communication with compartment 14K of the drinking-glass-like element 14 open at the top part in 14H and adapted to receive a mouthpiece member (of the known type and comprising a filter and an actual mouthpiece) or other similar element to carry out a test with the mouth or an instrument of the standard type so as to allow the known "sniff" test, not shown, required for the use of the device subject of the invention.

It should be observed that compared to a mouth test, the sniff test solely requires a change of the patient "interface". As a matter of fact, the test carried out with the mouth requires a mouthpiece member (or simply a mouthpiece), the sniffing one partly made of rubber (usually medical silicone) inserted into the nostril and connected to the MIP/MEP device through a flexible tube which is connected in the same point into which the mouthpiece is inserted. The terminal part of the tube requires a part made of rubber for adapting to the diameter of the opening into which the mouthpiece is inserted.

In the bottom wall 18A of the drinking-glass-like portion 18 there are present two curved elongated slots or holes 21 and 22 adapted to accommodate feet 24 protruding from the bottom 14P of the drinking-glass-like element 14 and adapted to be inserted and fixed in seats 25 provided for at the first end 11 of the cylindrical element 10. Therefore, given that the drinking-glass-like element 14 fixed to the cylindrical element 10 of the body 9, the switch element 16 may rotate around the axis W guided by the feet 24 in the slots 21 and 22; the length of said slots limits the rotation of the switch element 16 around the axis W.

The tubular part 17 is therefore adapted to receive from a patient air expired from the latter and be passed through by the air that such patient inspires (through the mouthpiece or another element for the through-flow of the air which is associated with the drinking-glass in a known manner 14).

The tubular part 17 has a through hole 28 in a wall 29 thereof (with axis K orthogonal to the axis W) adapted to place the hole 17K in communication with an opening 30 or 31 provided for in a cylindrical inner part 34 of the cylindrical element 10, that is tubular-shaped and which houses the tubular part 17 of the switch element 16 (and in which such tubular part can rotate).

Each hole 30 or 31 opens frontally at a corresponding first and second hole 36 and 37 obtained in the outer wall 13 of the cylindrical element 10 and which communicate with the external wall of the inner cavity 10A of such element 10 and therefore of the body 9 of the first component 2. Such holes 36 and 37 are arranged distant from each other, for example at 120° between each other.

In the first hole 36 there is arranged a check valve 38 adapted to allow the air inspired by the patient to pass through such first hole 36 and reach the hole 17K of the tubular part 17 and therefore the user when such hole 36 is coaxial with the opening 30 and the hole 28 mentioned above. In the second hole 37 instead, there is arranged a corresponding check valve 40 adapted to allow a flow of air expired by the patient which passes through in the hole 17K, through the holes 28 and 31 (when all coaxial) and therefore flows out to the external of the body 9 from the hole 37. Such flows are obtained by suitably rotating the switch element 16 as described below.

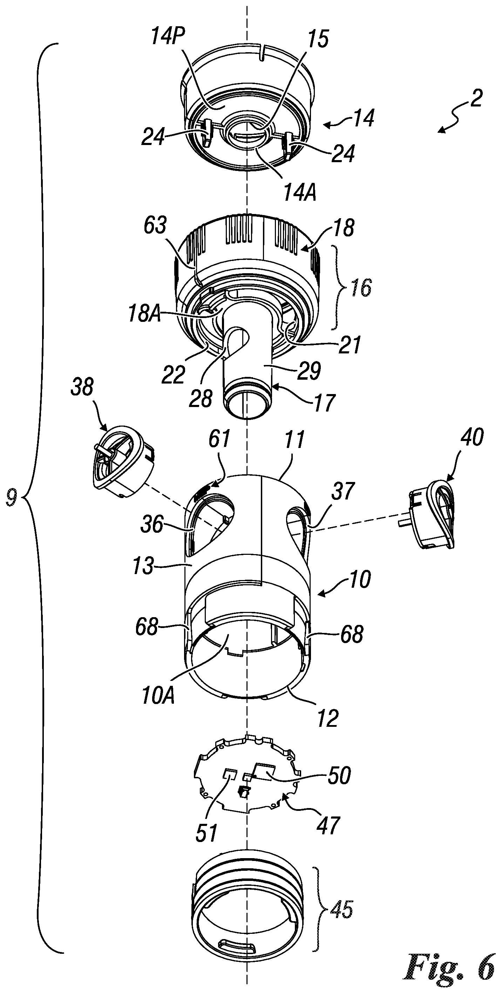

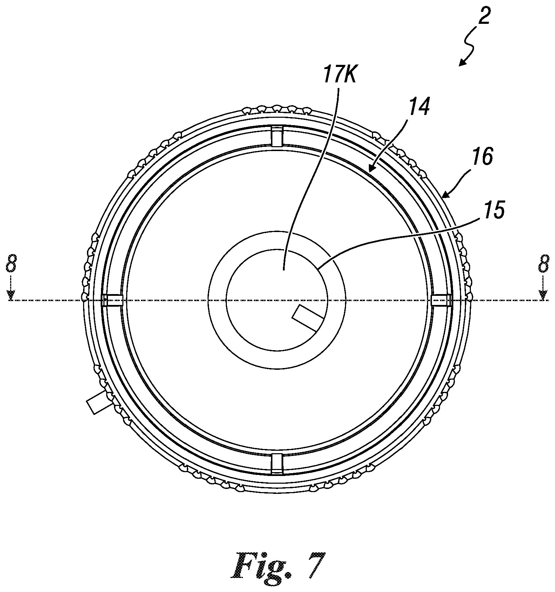

The cavity 10 is closed at the lower part by a closing element 45 inserted into the second end 12 of the cylindrical element 10 of the body 9. Such closing element 45 comprises a recessed seat 46, facing towards the cavity 10A and containing a support 47 for a circuit board 48 comprising a pressure transducer and an RFID TAG of the known type (indicated with 50 and 51 in figures 6 to 8 by way of example). Such RFID device is therefore fitted, fixed, directly in the circuit board 48 on the support 47, in the body 9.

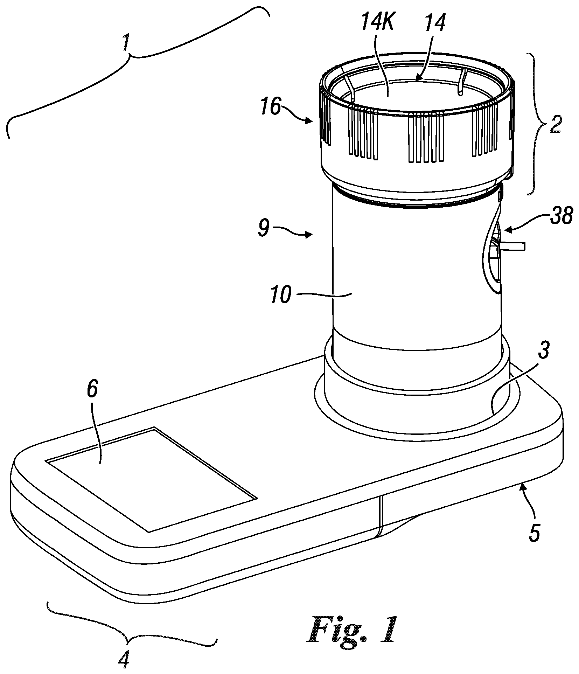

When using the device 1 it has the configuration of figure 1. Upon the request of the patient (who activates the device 1 in any known manner by acting on the second component 4), an RFID signal is sent to the pressure transducer which prepares to carry out the measurement.

When the patient inspires air and expires air through the tubular part 17 of the switch element 16, there is generated a vacuum/pressure above the support 47 which is detected by the circuit board 48 and in particular by the prior art pressure transducer 50. This allows said circuit board 48 to detect the value of the maximum inspired pressure (MIP) of the maximum expired pressure (MEP).

Through the RFID TAG, interrogated by the detector of the second component 4 mentioned above, the circuit board 48, allows the latter to know such measured value and show it on the display 6.

Furthermore, it should be observed that the position of the through hole 28 (movable given that it is associated with the tubular part 17 in turn component of the switch element 16 capable of rotating around the axis W) with respect to the openings 30 and 31 and to the holes 36, 37 (fixed) may be identified and defined in any known manner so as to facilitate the use of the device subject of the present invention. For example, the holes 36 and 37 provided with the respective check valves 38 and 40 and adapted to allow an MIP and MEP measurement can be identified with an appropriate wording 60 and 61 on the external of the wall 13 of the cylindrical element 10; a highlighter, for example a protruding notch 63, can be present on the external 66 of the drinking-glass-like portion 18 on which the patient intervened to obtain the rotation of the switch element 16. Bringing such notch 63 above the wordings 60 and 61, the patient easily and visually identifies the measurement (MIP and MEP) which can be carried out.

Furthermore, the closing element 45 is externally provided with L-shaped recesses 68 adapted to allow the coupling of the first component 2 in the seat 3 del secondo component 4, a coupling that is advantageously of the bayonet type.

Herein described is a preferred embodiment of the invention. However, others may be provided for: for example, the second component 4 may be without the display 6, but it may be connected from a remote position (by wi-fi, Bluetooth or the like) to an external device which evaluates data on the measurements carried out and displays them on its display.

The description outlined above and the variants mentioned above have the innovative characteristics of the present invention as reported in the claims below.

## Claims

1. Device for measuring the inspiration and expiration muscle strength of a patient by measuring a pressure of the air inspired and/or of the air expired by the patient, said device comprising a first tubular component (2) for measuring said pressure and a second component (4) to which the first component (2) is removably constrained, and adapted to read the measurements carried out by the first component (2) so that they can be indicated to the patient, said first component (2) having two openings (36, 37) provided with check valves (38, 40) adapted to respectively allow the flow of the inspired air and the flow of the of the expired air, said first component (2) having a tubular-shaped body (9), **characterised in that** said first component (2) comprises a switch element (16) rotatable around a longitudinal axis (W) of the tubular-shaped body (9), and adapted to allow the patient to alternatively inspire and expire the air from said valves (38, 40), said switch element comprising a control portion (18) arranged at a first end (11) of said tubular-shaped body (9) and connected to a tubular part (17) inside said body (9), the tubular part (17) comprising a hole (17K) open at its ends (17A, 17B) and laterally delimited by a wall (29) in which there is made a through hole (28) adapted to selectively cooperate with one of the openings (36, 37) provided with check valves (38, 40) following the rotation of the switching element (16) around said longitudinal axis, said openings being provided for in a wall (13) of said tubular-shaped body (9), said body (9) having an inner cavity (10A) in which there can be selectively rotated the tubular part (17) of the switch element (16), in said inner cavity (10A) there being provided for a pressure transducer (50) adapted to detect the maximum pressure of the inspired air (MIP) and the pressure of the expired air (MEP) through each of said openings (46, 47) in the wall (13) of the tubular-shaped body (9).

2. Device according to claim 1, **characterised in that** said pressure transducer (50) is part of a circuit board (48) fixed in said body (9) of the first tubular component (2), said circuit board comprising an RFID TAG adapted to transfer the detected maximum pressure datum (MIP, MEP) to a circuit board of the second component (4) which evaluates the maximum inspired pressure (MIP) and maximum expired pressure (MEP) and it allows to display the corresponding data.

3. Device according to claim 2, **characterised in that** the second component (4) comprises a display (6) on which there is displayed the maximum inspired and expired pressure.

4. Device according to claim 2, **characterised in** the inspired and expired pressure datum is displayed on a remote device to which there is connected the second component (4).

5. Device according to claim 1, **characterised in that** said switch element (16) rotates around the longitudinal axis (W) in a guided manner by a drinking-glass-like element (14) partially contained in the control portion (18), but having at least one protrusion (24) fixed to the cylindrical-shaped body (9) and inserted passing through into a slot (21, 22) provided for in a bottom wall (18A) of such control portion, said slot limiting the rotation of the switch element (16) around the longitudinal axis (W).

6. Device according to claim 5, **characterised in that** each protrusion (24) of the drinking-glass-like element (14) is fixed in a corresponding seat (25) provided for at the first end (11) of the tubular-shaped body (9).

7. Device according to claim 1, **characterised in that** the tubular-shaped body (9) comprises a cylindrical element (10) open at two opposite ends (11, 12), one of such ends defining the first end (11) of the tubular-shaped body (9), an outer wall of said cylindrical element (10) defining the wall (13) of said body (9) provided with the openings (36, 37) having the check valves (38, 40), said wall delimiting the inner cavity (10A) of said tubular-shaped body (9), the second end (12) of said cylindrical element (10) being enclosed by a closing element (45) with which there is functionally associated the pressure transducer (50).

8. Device according to claims 2 and 7, **characterised in that** said closing element (45) supports the circuit board (48) comprising the RFID TAG.

9. Device according to claim 7, **characterised in that** said cylindrical element (10) comprises an inner cylindrical part (34) in which it rotates the tubular part (17) of the switch element (16), said inner cylindrical part (34) comprising openings (30, 31) opening frontally to the openings (36,37) provided with check valves (38, 40).

10. Device according to claim 5, **characterized in that** it comprises a mouthpiece or a tool for carrying out the sniff test associated with the drinking-glass-like element (14).

11. Device according to claim 1, **characterised in that** the second component (4) is a component of a spirometer, a seat (3) of the latter adapted to contain a turbine for carrying out a spirometer test being used to contain the first component (2) of the device (1).

## Patentansprüche

1. Vorrichtung zum Messen der Ein- und Ausatmungsmuskelkraft eines Patienten durch Messen eines Drucks der Luft, die von dem Patienten eingeatmet und/oder ausgeatmet wird, die Vorrichtung umfassend eine erste rohrförmige Komponente (2) zum Messen des Drucks und eine zweite Komponente (4), an der die erste Komponente (2) lösbar befestigt ist, und angepasst ist, die Messungen auszulesen, die von der ersten Komponente (2) durchgeführt werden, damit sie dem Patienten angegeben werden können, wobei die erste Komponente (2) zwei Öffnungen (36, 37) aufweist, die mit Rückschlagventilen (38, 40) versehen sind, die angepasst sind, um jeweils den Fluss der eingeatmeten Luft und den Fluss der ausgeatmeten Luft zu ermöglichen, wobei die erste Komponente (2) einen rohrförmigen Körper (9) aufweist, **dadurch gekennzeichnet, dass** die erste Komponente (2) ein Schaltelement (16) umfasst, das um eine Längsachse (W) des rohrförmigen Körpers (9) drehbar und angepasst ist, um dem Patienten zu ermöglichen, abwechselnd Luft aus den Ventilen (38, 40) ein- und auszuatmen, das Schaltelement umfassend einen Steuerabschnitt (18), der an einem ersten Ende (11) des rohrförmigen Körpers (9) angeordnet und mit einem rohrförmigen Teil (17) innerhalb des Körpers (9) verbunden ist, das rohrförmige Teil (17) umfassend ein Loch (17K), das an seinen Enden (17A, 17B) offen und seitlich durch eine Wand (29) begrenzt ist, in der ein Durchgangsloch (28) ausgefertigt ist, das angepasst ist, um nach der Drehung des Schaltelements (16) um die Längsachse selektiv mit einer der Öffnungen (36, 37) zusammenzuwirken, die mit Rückschlagventilen (38, 40) versehen sind, wobei die Öffnungen in einer Wand (13) des rohrförmigen Körpers (9) bereitgestellt sind, der Körper (9) einen inneren Hohlraum (10A) aufweist, in dem das rohrförmige Teil (17) des Schaltelements (16) selektiv gedreht werden kann, wobei in dem inneren Hohlraum (10A) ein Druckwandler (50) bereitgestellt ist, der angepasst ist, um den maximalen Druck der eingeatmeten Luft (MIP) und den Druck der ausgeatmeten Luft (MEP) über jede der Öffnungen (46, 47) in der Wand (13) des rohrförmigen Körpers (9) zu erfassen.

2. Vorrichtung nach Anspruch 1, **dadurch gekennzeichnet, dass** der Druckwandler (50) Teil einer Leiterplatte (48) ist, die in de Körper (9) der ersten rohrförmigen Komponente (2) befestigt ist, die Leiterplatte umfassend einen RFID-Tag, der angepasst ist, um die erfassten maximalen Daten (MIP, MEP) an eine Leiterplatte der zweiten Komponente (4) zu übertragen, die den maximalen Einatmungsdruck (MIP) und den maximalen Ausatmungsdruck (MEP) auswertet und ein Anzeigen der entsprechenden Daten ermöglicht.

3. Vorrichtung nach Anspruch 2, **dadurch gekennzeichnet, dass** die zweite Komponente (4) eine Anzeige (6) umfasst, auf der der maximale Ein- und Ausatmungsdruck angezeigt wird.

4. Vorrichtung nach Anspruch 2, **dadurch gekennzeichnet, dass** die Daten des Ein- und Ausatmungsdrucks auf einer entfernten Vorrichtung angezeigt werden, mit der die zweite Komponente (4) verbunden ist.

5. Vorrichtung nach Anspruch 1, **dadurch gekennzeichnet, dass** das Schaltelement (16) auf eine durch ein trinkglasartiges Element (14) geführte Weise, das teilweise in dem Steuerabschnitt (18) enthalten ist, um die Längsachse (W) dreht, das jedoch mindestens einen Vorsprung (24) aufweist, der an dem zylinderförmigen Körper (9) befestigt ist und durch einen in einer Bodenwand (18A) eines solchen Steuerabschnitts bereitgestellten Schlitz (21, 22) hindurchgeführt ist, wobei der Schlitz die Drehung des Schaltelements (16) um die Längsachse (W) begrenzt.

6. Vorrichtung nach Anspruch 5, **dadurch gekennzeichnet, dass** jeder Vorsprung (24) des trinkglasartigen Elements (14) in einem entsprechenden Sitz (25) befestigt ist, der an dem ersten Ende (11) des rohrförmigen Körpers (9) bereitgestellt ist.

7. Vorrichtung nach Anspruch 1, **dadurch gekennzeichnet, dass** der rohrförmige Körper (9) ein zylindrisches Element (10) umfasst, das an zwei gegenüberliegenden Enden (11, 12) offen ist, eines dieser Enden das erste Ende (11) des rohrförmigen Körpers (9) definiert, eine Außenwand des zylindrischen Elements (10) die die Wand (13) des Körpers (9) definiert, der mit den Öffnungen (36, 37) versehen ist, die die Rückschlagventile (38, 40) aufweisen, die Wand den inneren Hohlraum (10A) des rohrförmigen Körpers (9) begrenzt, das zweite Ende (12) des zylindrischen Elements (10) von einem Verschlusselement (45) umschlossen ist, mit dem der Druckwandler (50) funktionsfähig assoziiert ist.

8. Vorrichtung nach den Ansprüchen 2 und 7, **dadurch gekennzeichnet, dass** das Verschlusselement (45) die Leiterplatte (48) trägt, die den RFID-TAG umfasst.

9. Vorrichtung nach Anspruch 7, **dadurch gekennzeichnet, dass** das zylindrische Element (10) einen inneren zylindrischen Teil (34) umfasst, in dem der rohrförmige Teil (17) des Schaltelements (16) dreht, wobei der innere zylindrische Teil (34) Öffnungen (30, 31) umfasst, die frontal zu den Öffnungen (36, 37) münden, die mit Rückschlagventilen (38, 40) versehen sind.

10. Vorrichtung nach Anspruch 5, **dadurch gekennzeichnet, dass** sie ein Mundstück oder ein Werkzeug zum Durchführung des Geruchstests umfasst, das mit dem trinkglasartigen Element (14) assoziiert ist.

11. Vorrichtung nach Anspruch 1, **dadurch gekennzeichnet, dass** die zweite Komponente (4) eine Komponente eines Spirometers ist, ein Sitz (3) letzteren angepasst ist, um eine Turbine zum Durchführen eines Spirometrie-Tests zu enthalten, die verwendet wird, um die erste Komponente (2) der Vorrichtung (1) zu enthalten.

## Revendications

1. Dispositif destiné à mesurer la force musculaire inspiratoire et expiratoire d'un patient en mesurant la pression de l'air inspiré et/ou de l'air expiré par le patient, ledit dispositif comprenant un premier composant tubulaire (2) destiné à mesurer ladite pression et un deuxième composant (4) auquel le premier composant (2) est fixé de manière amovible, et adapté pour lire les mesures effectuées par le premier composant (2) de manière à ce qu'elles puissent être indiquées au patient, ledit premier composant (2) comportant deux ouvertures (36, 37) munies de clapets anti-retour (38, 40) adaptés pour permettre respectivement l'écoulement de l'air inspiré et l'écoulement de l'air expiré, ledit premier composant (2) comportant un corps de forme tubulaire (9), **caractérisé en ce que** ledit premier composant (2) comprend un élément de commutation (16) pouvant tourner autour d'un axe longitudinal (W) du corps de forme tubulaire (9), et adapté pour permettre au patient d'inspirer et d'expirer alternativement l'air par lesdits clapets (38, 40), ledit élément de commutation comprenant une partie de commande (18) disposée à une première extrémité (11) dudit corps de forme tubulaire (9) et reliée à une partie tubulaire (17) à l'intérieur dudit corps (9), la partie tubulaire (17) comportant un trou (17K) ouvert à ses extrémités (17A, 17B) et délimité latéralement par une paroi (29) dans laquelle est pratiqué un trou débouchant (28) adapté pour coopérer sélectivement avec l'une des ouvertures (36, 37) munies de clapets anti-retour (38, 40) suite à la rotation de l'élément de commutation (16) autour dudit axe longitudinal, lesdites ouvertures étant prévues dans une paroi (13) dudit corps de forme tubulaire (9), ledit corps (9) comportant une cavité interne (10A) dans laquelle peut tourner de manière sélective la partie tubulaire (17) de l'élément de commutation (16), dans ladite cavité interne (10A) étant prévu un transducteur de pression (50) adapté pour détecter la pression maximale de l'air inspiré (MIP) et la pression de l'air expiré (MEP) à travers chacune desdites ouvertures (46, 47) dans la paroi (13) du corps de forme tubulaire (9).

2. Dispositif selon la revendication 1, **caractérisé en ce que** ledit transducteur de pression (50) fait partie d'un circuit imprimé (48) fixé dans ledit corps (9) du premier composant tubulaire (2), ledit circuit imprimé comprenant une étiquette RFID adaptée pour transférer les données de pression maximale détectées (MIP, MEP) à un circuit imprimé du deuxième composant (4) qui évalue la pression inspiratoire maximale (MIP) et la pression expiratoire maximale (MEP) et permet d'afficher les données correspondantes.

3. Dispositif selon la revendication 2, **caractérisé en ce que** le deuxième composant (4) comprend un écran (6) sur lequel s'affichent les pressions inspiratoires et expiratoires maximales.

4. Dispositif selon la revendication 2, **caractérisé en ce que** les données relatives à la pression inspiratoire et expiratoire sont affichées sur un dispositif distant auquel est connecté le deuxième composant (4).

5. Dispositif selon la revendication 1, **caractérisé en ce que** ledit élément de commutation (16) tourne de manière guidée autour de l'axe longitudinal (W) grâce à un élément en forme de verre à boire (14) partiellement logé dans la partie de commande (18), mais comportant au moins une saillie (24) fixée au corps de forme cylindrique (9) et insérée de manière traversante dans une fente (21, 22) prévue dans une paroi inférieure (18A) de ladite partie de commande, ladite fente limitant la rotation de l'élément de commutation (16) autour de l'axe longitudinal (W).

6. Dispositif selon la revendication 5, **caractérisé en ce que** chaque saillie (24) de l'élément en forme de verre à boire (14) est fixée dans un logement correspondant (25) prévu à la première extrémité (11) du corps de forme tubulaire (9).

7. Dispositif selon la revendication 1, **caractérisé en ce que** le corps de forme tubulaire (9) comprend un élément cylindrique (10) ouvert à deux extrémités opposées (11, 12), l'une de ces extrémités définissant la première extrémité (11) du corps de forme tubulaire (9), une paroi extérieure dudit élément cylindrique (10) définissant la paroi (13) dudit corps (9) pourvue des ouvertures (36, 37) comportant les clapets anti-retour (38, 40), ladite paroi délimitant la cavité interne (10A) dudit corps de forme tubulaire (9), la deuxième extrémité (12) dudit élément cylindrique (10) étant fermée par un élément de fermeture (45) auquel est associé fonctionnellement le transducteur de pression (50).

8. Dispositif selon les revendications 2 et 7, **caractérisé en ce que** ledit élément de fermeture (45) supporte le circuit imprimé (48) comprenant l'étiquette RFID.

9. Dispositif selon la revendication 7, **caractérisé en ce que** ledit élément cylindrique (10) comprend une partie cylindrique interne (34) dans laquelle tourne la partie tubulaire (17) de l'élément de commutation (16), ladite partie cylindrique interne (34) comportant des ouvertures (30, 31) s'ouvrant frontalement sur les ouvertures (36, 37) munies de clapets anti-retour (38, 40).

10. Dispositif selon la revendication 5, **caractérisé en ce qu'**il comprend un embout buccal ou un outil permettant d'effectuer le test olfactif, associé à l'élément en forme de verre à boire (14).

11. Dispositif selon la revendication 1, **caractérisé en ce que** le deuxième composant (4) est un composant d'un spiromètre, un logement (3) de ce dernier, conçu pour recevoir une turbine destinée à effectuer un test spirométrique, étant utilisé pour recevoir le premier composant (2) du dispositif (1).
